# EUROPEAN PATENT SPECIFICATION

(11) **EP 4 537 892 B1**
(45) Date of publication and mention of the grant of the patent: **06.05.2026**
(21) Application number: 22945283.4
(22) Date of filing: 09.06.2022
(51) Int. Cl.: A61N 1/39

(54) **MEDICAL DEVICE WITH DEFIBRILLATION FUNCTION**
MEDIZINISCHE VORRICHTUNG MIT DEFIBRILLATIONSFUNKTION
DISPOSITIF MÉDICAL AVEC FONCTION DE DÉFIBRILLATION

(43) Date of publication of application: 16.04.2025
(73) Proprietor: Shenzhen Mindray Bio-Medical Electronics Co., Ltd., Shenzhen, Guangdong 518057 (CN)
(72) Inventor: YANG, Ping, Shenzhen, Guangdong 518057 (CN); CHEN, Dabing, Shenzhen, Guangdong 518057 (CN); HE, Xianliang, Shenzhen, Guangdong 518057 (CN); JIANG, Haoyu, Shenzhen, Guangdong 518057 (CN)
(74) Representative: KIPA AB
(86) International application number: PCT/CN2022/097829
(87) International publication number: WO 2023/236138

(56) References cited:
- CN-A- 104 203 138
- CN-A- 110 812 699
- CN-A- 111 529 939
- CN-A- 112 022 107
- CN-A- 113 244 534
- CN-U- 213 941 876
- US-A1- 2014 371 806
- US-A1- 2018 280 709
- US-A1- 2020 253 820
- US-B1- 10 625 088

## Description

### TECHNICAL FIELD

The disclosure relates to the technical field of medical device, and more specifically, to a medical device with a defibrillator function.

### BACKGROUND

Heart disease, such as Out-of-Hospital Cardiac Arrest (OHCA), is one of leading causes of death in humans. A large proportion of Out-of-Hospital Cardiac Arrests are attributed to ventricular fibrillation or pulseless ventricular tachycardia (VF/VT). If timely defibrillation is available, these patients will have a higher survival rate.

Although significant progress has been made in external defibrillation technology, conventional defibrillators only provide single-channel defibrillation, which urgently needs to improve the defibrillation effect for refractory ventricular fibrillation. Refractory ventricular fibrillation refers that a heart rhythm of the patient still remains VF/VT but fails to be converted to a sustained organized rhythm, after multiple (>3) attempts of conventional defibrillation. About 10% of patients with Out-of-Hospital Cardiac Arrest of ventricular fibrillation suffer from refractory ventricular fibrillation, while conventional single-channel defibrillation technology has difficulty in restoring organized rhythm for such patients in a timely manner.

US 10625088 discloses a medical device with a defibrillation function.

### SUMMARY

The invention is defined by the independent claim 1. This disclosure is provided to solve above-mentioned problem(s) in the prior art.

This disclosure requires a medical device with a defibrillator function, a method for configuring a medical device with a defibrillator function, and a medium, which are capable of providing an open architecture and function, flexibly and efficiently satisfying defibrillation requirements of cardiac arrhythmias in different situations, and are also relatively convenient to use.

According to a first aspect of this disclosure, a medical device with a defibrillation function is provided. The medical device includes a host, which is capable of being assembled with at least one defibrillation module, which assembly is capable of being disassembled, such that at least two defibrillation modules are capable of being combined to the medical device. The host is preset with a single-channel defibrillation mode which supports a single defibrillation module to perform a defibrillation operation, and a dual-channel defibrillation mode which supports two defibrillation modules to cooperatively perform a defibrillation operation. The host includes a processor, which is configured to: detect a number of defibrillation module(s) which is(are) included by the medical device; determine the single-channel defibrillation mode as a target defibrillation mode of the medical device when a single defibrillation module is detected, and control the medical device to perform a defibrillation operation in said target defibrillation mode; automatically determine the single-channel defibrillation mode or the dual-channel defibrillation mode, or select one from the single-channel defibrillation mode and the dual-channel defibrillation mode based on a selection instruction of a user, as a target defibrillation mode of the medical device, when two defibrillation modules are detected; and control the medical device to perform a defibrillation operation in said target defibrillation mode.

According to a second non-claimed aspect of this disclosure, a method for configuring a medical device with a defibrillation function is provided.

The method includes following steps. Provide a host and at least one defibrillation module which is capable of being assembled to the host, which assembly is capable of being disassembled. Preset the host with a single-channel defibrillation mode which supports a single defibrillation module to perform a defibrillation operation, and a dual-channel defibrillation mode which supports two defibrillation modules to cooperatively perform a defibrillation operation. Detect a number of defibrillation module(s) which is(are) included by the medical device. Determine the single-channel defibrillation mode as a target defibrillation mode of the medical device when a single defibrillation module is detected, and control the medical device to perform a defibrillation operation in said target defibrillation mode. Automatically determine the single-channel defibrillation mode or the dual-channel defibrillation mode, or select one from the single-channel defibrillation mode and the dual-channel defibrillation mode based on a selection instruction of a user, as a target defibrillation mode of the medical device, when two defibrillation modules are detected; and control the medical device to perform a defibrillation operation in said target defibrillation mode.

According to a third aspect of this disclosure, a medical device with a defibrillation function is provided. The medical device includes a host which is provided with a processor. The host is capable of being assembled with at least one defibrillation module, which assembly is capable of being disassembled, such that at least N defibrillation modules are capable of being combined to the medical device. The host is preset with defibrillation modes which correspond to the defibrillation module(s), wherein the defibrillation modes include a single-channel defibrillation mode which supports a single defibrillation module to perform a defibrillation operation, and a K-channel defibrillation mode which supports K defibrillation modules to cooperatively perform a defibrillation operation. The processor is configured to: detect a number of defibrillation module(s) which is(are) included by the medical device; determine the single-channel defibrillation mode as a target defibrillation mode of the medical device when a single defibrillation module is detected, and control the medical device to perform a defibrillation operation in said target defibrillation mode; automatically determine the single-channel defibrillation mode or the K-channel defibrillation mode, or select one from the single-channel defibrillation mode and the K-channel defibrillation mode based on a selection instruction of a user, as a target defibrillation mode of the medical device, when N defibrillation modules are detected, and control the medical device to perform a defibrillation operation in said target defibrillation mode. Wherein, K is greater than 1, but less than or equal to N, K and N are both integers.

According to a fourth non-claimed aspect of this disclosure, a method for configuring a medical device with a defibrillation function is provided. The method includes following steps. Provide a host and at least one defibrillation module which is capable of being assembled to the host, which assembly is capable of being disassembled. Preset the host with defibrillation modes which correspond to the defibrillation module(s), wherein the defibrillation modes include a single-channel defibrillation mode which supports a single defibrillation module to perform a defibrillation operation, and a K-channel defibrillation mode which supports K defibrillation modules to cooperatively perform a defibrillation operation. Detect a number of defibrillation module(s) which is(are) by the medical device. Determine the single-channel defibrillation mode as a target defibrillation mode of the medical device when a single defibrillation module is detected, and control the medical device to perform a defibrillation operation in said target defibrillation mode. Automatically determine the single-channel defibrillation mode or the K-channel defibrillation mode, or select one from the single-channel defibrillation mode and the K-channel defibrillation mode based on a selection instruction of a user, as a target defibrillation mode of the medical device, when N defibrillation modules are detected, and control the medical device to perform a defibrillation operation in said target defibrillation mode. Wherein, K is greater than 1, but less than or equal to N, K and N are both integers.

According to a fifth non-claimed aspect of this disclosure, a computer-readable storage medium is provided, on which computer program instructions are stored. The computer program instructions implement a method for configuring when executed by a host of a medical device with a defibrillation function. The host is capable of being assembled with at least one defibrillation module, which assembly is capable of being disassembled; such that at least two defibrillation modules are capable of being combined to the medical device. The method includes following steps. Preset the host with defibrillation modes which correspond to the defibrillation module(s), wherein the defibrillation modes include a single-channel defibrillation mode which supports a single defibrillation module to perform a defibrillation operation, and a dual-channel defibrillation mode which supports two defibrillation modules to cooperatively perform a defibrillation operation. Detect a number of defibrillation module(s) which is(are) included by the medical device. Determine the single-channel defibrillation mode as a target defibrillation mode of the medical device when a single defibrillation module is detected, and control the medical device to perform a defibrillation operation in said target defibrillation mode. Automatically determine the single-channel defibrillation mode or the dual-channel defibrillation mode, or select one from the single-channel defibrillation mode and the dual-channel defibrillation mode based on a selection instruction of a user, as a target defibrillation mode of the medical device, when two defibrillation modules are detected; and control the medical device to perform a defibrillation operation in said target defibrillation mode.

The medical device with a defibrillator function, the non-claimed method for configuring a medical device with a defibrillator function, and the non-claimed medium are capable of providing an open architecture and function, flexibly and efficiently satisfying defibrillation requirements of cardiac arrhythmias in different situations (for example, but not limited to Out-of-Hospital Cardiac Arrest), such as a requirement for single-channel defibrillation, a requirement for dual-channel defibrillation, a requirement for multi-channel defibrillation, etc., thereby combining a desired number of defibrillator modules at the medical device by assembling at least one defibrillation module to the host, which assembly is capable of being disassembled. Furthermore, the host is capable of automatically detecting a number of combined defibrillation module(s) and adapt a target defibrillation mode, so as to enable the medical device to perform a defibrillation operation in said target defibrillation mode, thereby flexibly and efficiently satisfying defibrillation requirements of cardiac arrhythmias in different situations, and also being more convenient to use. In addition, this open disassembly and assembly (such as but not limited to plug-in) design significantly reduces a maintenance cost of the medical device. For example, when a defibrillator module fails, there is no need to scrap the host. The required defibrillation operation can be restored by replacing the failed one with another defibrillator module that is working normally and assembling said defibrillator module to the host.

### BRIEF DESCRIPTION OF THE DRAWINGS

In the drawings, which are not necessarily drawn to scale, same reference numbers may describe similar components in different views. The same reference numerals with or without a different letter suffix may represent similar components of different instances. The drawings illustrate various embodiments generally by way of example rather than limitation, and serve to explain the disclosed embodiments together with the description and claims. Such embodiments are illustrative and are not intended to be exhaustive or exclusive embodiments of the present device or method.
FIG. 1 is a diagram of a medical device with a defibrillation function according to an embodiment of this disclosure.
FIG. 2 is a flowchart of a non-claimed method for configuring a medical device with a defibrillation function according to an embodiment of this disclosure.
FIG. 3 is a diagram of a first interface for selecting a defibrillation module of a medical device with a defibrillation function according to an embodiment of this disclosure.
FIG. 4 is a diagram of a second interface for selecting a defibrillation module of a medical device with a defibrillation function according to an embodiment of this disclosure.
FIG. 5 is a diagram of a medical device with a defibrillation function according to an embodiment of this disclosure.
FIG. 6 is a flowchart of a non-claimed method for configuring a medical device with a defibrillation function according to an embodiment of this disclosure.
FIG. 7 is a diagram of a multi-channel discharge waveform when a multi-channel sequential external defibrillation mode is adopted according to an embodiment of this disclosure.
FIG. 8 is a structural block diagram of a medical device with a defibrillation function according to an embodiment of this disclosure.

### DESCRIPTION OF THE PREFERRED EMBODIMENTS

In order to enable those skilled in the art to better understand the technical solution of this disclosure, this disclosure is described in detail below in conjunction with the accompanying drawings and specific implementation methods. The embodiments of this disclosure are further described in detail below in conjunction with the drawings and specific embodiments, but are not intended to limit this disclosure. For the steps described in this disclosure, if there is no necessary causal relationship between each other, the order in which they are described as examples in this disclosure should not be considered as a limitation. Those skilled in the art should know that the order can be adjusted as long as the logic between them is not destroyed to make the entire process cannot be implemented.

In addition, those skilled in the art will appreciate that the drawings provided herein are for illustration purposes and are not necessarily drawn to scale.

Unless the context clearly requires otherwise, throughout the description and claims, the words "include", "including" and similar words should be interpreted in an inclusive sense rather than an exclusive or exhaustive sense; that is, in the sense of "including but not limited to". In the description of this disclosure, unless otherwise specified, "multiple" means two or more.

Specifically, defibrillation refers to a process of terminating ventricular fibrillation (VF) using a medical device or specific drugs. Defibrillation can be divided into three categories according to different methods, namely, electrical defibrillation that terminates ventricular fibrillation by shocking the heart with a certain amount of electric current, drug defibrillation that defibrillates by injecting drugs to affect the electrical activity of the heart, and manual defibrillation by tapping the precordial area. In medicine, "defibrillation" usually refers specifically to an operation of using a defibrillator to discharge a current to the heart to terminate atrial or ventricular fibrillation. The "defibrillation function" of the medical device in this disclosure is intended to mean a use of a strong pulse current through the heart to eliminate arrhythmia and restore sinus rhythm.

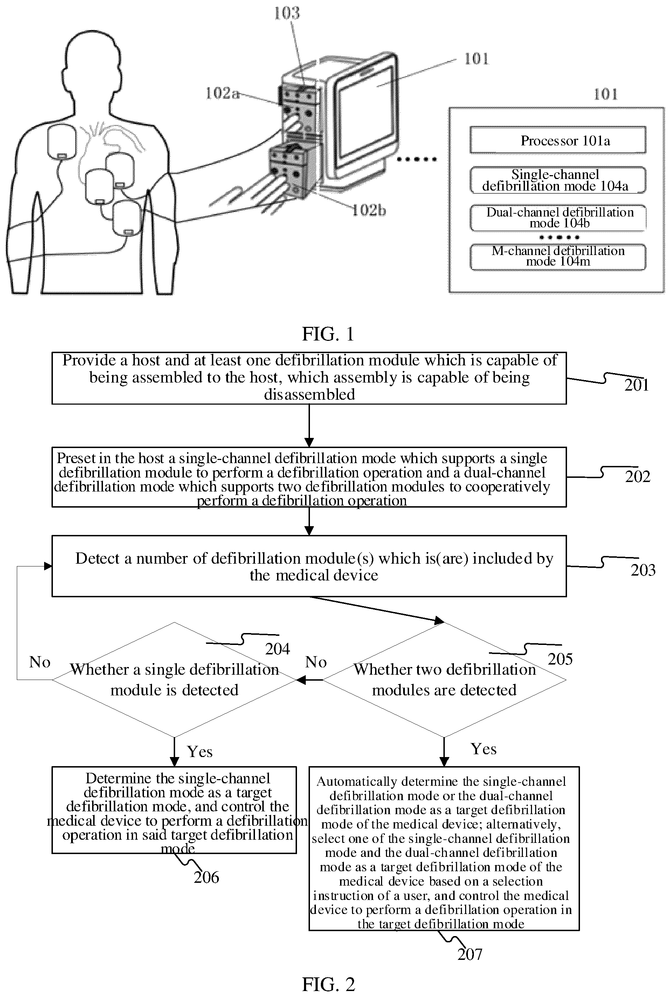

FIG. 1 is a diagram of a medical device with a defibrillation function according to an embodiment of this disclosure. As shown in FIG. 1, the medical device with a defibrillation function includes a host 101, which is capable of being assembled with at least one defibrillation module, which assembly is capable of being disassembled, such that at least two defibrillation modules are capable of being combined to the medical device. It is noted that, although FIG. 1 shows two assembled defibrillator modules 102a and 102b as an example, but the at least two defibrillator modules combined to the medical device do not necessarily have to be assembled externally, but can be built-in modules of the medical device. For example, a defibrillator module may be a built-in module inside the medical device, and the other defibrillator module may be assembled from an external source, so that at least two defibrillator modules can be combined therein. The medical device referred to in this disclosure as "combines" or "includes" a defibrillator module (or another module) may be internally fixed with or externally assembled with the defibrillator module. In the following, unless the defibrillation modules are distinguished from each other, they are all collectively denoted by reference numeral 102.

The host 101 is preset with a single-channel defibrillation mode 104a which supports a single defibrillation module to perform a defibrillation operation, and a dual-channel defibrillation mode 104b which supports two defibrillation modules to cooperatively perform a defibrillation operation.

The single-channel defibrillation mode 104a and the dual-channel defibrillation mode 104b may be preset in various ways. For example, the host 101 may pre-store control processes (or executable instructions) in various defibrillation modes, which define how to schedule and control related components to cooperatively perform a process flow of the defibrillation operation in these defibrillation modes. The process flow is not limited to being preset in software, but may also be preset in hardware. For example, by presetting in hardware, the defibrillation mode of each channel may be equipped with a corresponding control circuit, such as but not limited to SOC, ASIC, etc., so that when the defibrillation mode to be adopted is determined, the corresponding control circuit can be enabled. The process flow can also be preset by both the hardware and software in combination. In some embodiments, the host 101 may have preset defibrillation mode(s) which is(are) associated with a number of the defibrillation module(s) 102 included in the medical device, such as a single-channel defibrillation mode which is associated with a single defibrillation module 102; a dual-channel defibrillation mode and a single-channel defibrillation mode which are associated with two defibrillation modules 102.

The host 101 includes a processor 101a, which is configured to perform the following steps. Detect a number of defibrillation module(s) 102 which is(are) included by the medical device. Determine the single-channel defibrillation mode 104a as a target defibrillation mode of the medical device when a single defibrillation module 102 is detected, and control the medical device to perform a defibrillation operation in said target defibrillation mode. Automatically determine the single-channel defibrillation mode 104a or the dual-channel defibrillation mode 104b, or select one from the single-channel defibrillation mode 104a and the dual-channel defibrillation mode 104b based on a selection instruction of a user, as a target defibrillation mode of the medical device, when two defibrillation modules 102 (such as the defibrillator module 102a and the defibrillator module 102b in drawings) are detected, and control the medical device to perform a defibrillation operation in said target defibrillation mode.

The processor 101a may be a processing device including one or more general-purpose processing devices, such as a microprocessor, a central processing unit (CPU), a graphics processing unit (GPU), and the like. More specifically, the processor may be a complex instruction set computing (CISC) microprocessor, a reduced instruction set computing (RISC) microprocessor, a very long instruction word (VLIW) microprocessor, a processor executing other instruction sets, or a processor executing a combination of instruction sets. The processor may also be one or more special-purpose processing devices, such as an application-specific integrated circuit (ASIC), a field-programmable gate array (FPGA), a digital signal processor (DSP), a system on a chip (SoC), etc.

In this way, the medical device provides an open architecture and function, which allows the user to assemble to the host 101 at least one defibrillator module 102 which assembly is capable of being disassembled, according to defibrillation requirements of cardiac arrhythmias in different situations (such as but not limited to Out-of-Hospital Cardiac Arrest), such as a defibrillation requirement for single-channel defibrillation, especially, a defibrillation requirement for dual-channel defibrillation for refractory ventricular fibrillation, etc., such that the desired number of defibrillator modules (such as at least two defibrillator modules required for the dual-channel defibrillation mode) are combined in the medical device. The user only needs to conveniently plug in the required defibrillator module or perform other detachable connection operation to the required defibrillator module, the host 101 can automatically in response to detect the number of combined defibrillator module(s) 102, and adapt a target defibrillation mode, so as to enable the medical device to perform the defibrillation operation in the target defibrillation mode, thereby flexibly and efficiently satisfying the defibrillation requirements of cardiac arrhythmias in different situations, and also being relatively convenient to use. In addition, this open plug-in design significantly reduces the maintenance cost of the medical device with a defibrillator function. For example, when the defibrillator module 102a fails, there is no need to scrap the host 101. The required defibrillation operation can be restored by replacing the failed one with another defibrillator module 102b that is working normally and assembling said defibrillator module 102b to the host 101.

In some embodiments, at least one accommodation cavity 103 may be provided at the host 101, and each defibrillator module 102 may be accommodated inside the accommodation cavity 103 in a plug-in manner, that is, the defibrillator module 102 may be conveniently plugged into or pulled out of the accommodation cavity 103. In this way, the medical device can be "customized" to the functional configuration desired by the user in a plug-and-play manner, and even a user with average professional level can use it correctly.

The medical device provided in this disclosure can provide a defibrillation function but is not limited thereto. In addition to being able to combine (include) at least two defibrillation modules 102, the host 101 can also be combined with medical modules with other functions (not shown), such as but not limited to a monitoring module. Similar to the defibrillator module 102, medical module(s) may be assembled at the host 101, which assembly is capable of being dissembled, or may be directly arranged inside the host 101. When the medical device includes a monitoring module, the processor 101a can control the monitoring module to perform a monitoring operation in a monitoring mode, so as to monitor physiological sign information of patient(s). The physiological sign information may include at least one of an electrocardiogram signal, pulse information, a blood oxygen signal, and a blood pressure. The medical device provided in this way can not only perform a defibrillation operation, but also perform effective physiological monitoring of a patient based on the physiological sign information which is monitored by the monitoring module, thereby being able to detect whether the patient has abnormalities such as arrhythmia, and what type of arrhythmia exists. In particular, by combining at least two defibrillation modules 102 and a monitoring module to one device, said device is capable of performing a defibrillation operation and a monitoring operation simultaneously. It is understandable that the defibrillation operation generally occurs in scenarios where emergency treatment is required, and medical staff may be under great psychological pressure and may be in a state of high mental tension. By integrating the defibrillator module 102 and the monitoring module into one device, medical staff only need to focus on one device. Furthermore, various physiological sign information can be presented on a display at the same time, so that when the user performs the defibrillation operation, the user can conveniently view a display screen on the display, and make reference of the physiological sign information, so as to free up hands of the user to perform the defibrillation operation and other first aid operations.

In some embodiments, the physiological sign information typically includes at least an electrocardiogram (ECG) signal. In this way, the processor 101a can control start and stop of a defibrillation operation of a corresponding defibrillation module 102 in the target defibrillation mode according to the electrocardiogram signal which is detected by the monitoring module. The causes of cardiac arrhythmia are different, and corresponding ECG signals are also different. Therefore, cause of the cardiac arrhythmia can be identified from a ECG signal, and the start and stop of the defibrillation operation of the corresponding defibrillation module 102 can be controlled in a target manner, so that the adopted defibrillation operation is suitable for the cardiac arrhythmia caused by said cause. For example, cardiac arrhythmia caused by cardiac arrest cannot be defibrillated, while cardiac arrhythmia caused by ventricular fibrillation can be defibrillated.

In some embodiments, as shown in FIG. 1, the preset defibrillation mode may further include an M-channel defibrillation mode 104m which supports M defibrillation modules to cooperatively perform a defibrillation operation, wherein M is an integer greater than 2. In order to provide the M-channel defibrillation mode 104m, accordingly, N defibrillation modules 102 need to be combined in the medical device, wherein N is an integer and is equal to or greater than M. The processor 101a is also configured to: automatically determine the single-channel defibrillation mode 104a, the dual-channel defibrillation mode 104b, or the M-channel defibrillation mode 104m as a target defibrillation mode of the medical device, or select one from the single-channel defibrillation mode 104a, the dual-channel defibrillation mode 104b, and the M-channel defibrillation mode 104m, based on a selection instruction of a user, as a target defibrillation mode of the medical device, when N defibrillation modules 102 are detected; and control the medical device to perform a defibrillation operation in the target defibrillation mode. In this way, the medical device can also plug and combine the required number of defibrillation modules 102 into the host 101 according to specific defibrillation requirements of out-of-hospital cardiac arrhythmias, such as a requirement for M-channel defibrillation mode 104m for refractory ventricular fibrillation or pulseless ventricular tachycardia, so as to enable the medical device to match with the desired target defibrillation mode and perform a defibrillation operation accordingly to eliminate cardiac fibrillation, such as refractory ventricular fibrillation or pulseless ventricular tachycardia.

FIG. 2 is a flowchart of a non-claimed method for configuring a medical device with a defibrillation function according to an embodiment of this disclosure. As shown in FIG. 2, the method may start at step 201, in which provide a host and at least one defibrillation module which is capable of being assembled to the host, which assembly is capable of being disassembled. In step 202, preset in the host a single-channel defibrillation mode which supports a single defibrillation module to perform a defibrillation operation and a dual-channel defibrillation mode which supports two defibrillation modules to cooperatively perform a defibrillation operation. Please note that there is no particular limitation on an execution order of step 201 and step 202 as long as they do not affect their respective implementations. Steps 201 and 202 are structure and setting configurations before an operation phase of the medical device with a defibrillation function. Furthermore, the medical device is configured for the operation phase. Specifically, in step 203, detect a number of defibrillation module(s) which is(are) included by the medical device. Determine whether two defibrillation modules are detected (step 205). If yes, automatically determine the single-channel defibrillation mode or the dual-channel defibrillation mode as a target defibrillation mode of the medical device; alternatively, select one of the single-channel defibrillation mode and the dual-channel defibrillation mode as a target defibrillation mode of the medical device based on a selection instruction of a user, and control the medical device to perform a defibrillation operation in the target defibrillation mode (step 207). If a determination result of step 205 is No, determine whether a single defibrillation module is detected (step 204). If yes, determine the single-channel defibrillation mode as a target defibrillation mode, and control the medical device to perform a defibrillation operation in said target defibrillation mode (step 206). In FIG. 2, firstly determine whether two defibrillator modules are detected, and then determine whether a single defibrillator module is detected, which can avoid redundant detection of the single defibrillator module, thereby improving process efficiency. However, this is only an example, and step 204 and step 205 may also be performed in parallel, as long as the detection and determination of the two defibrillation modules together with the detection and determination of the single defibrillation module are finally achieved. If no single defibrillator module is detected (the determination result of step 204 is No) or no two defibrillator modules are detected (the determination result of step 205 is No), the process returns to step 203 to continue scanning and detecting whether the medical device includes a new defibrillator module.

Now returning to FIG. 1, details of the operation phase are specifically described in conjunction with the construction of the medical device. Note that various technical details of the medical device with a defibrillation function and the method for configuring a medical device with a defibrillation function described in various embodiments of this disclosure can be combined individually or in combination with the embodiment shown in FIG. 2, and are not repeated here.

For example, the defibrillation mode also includes an M-channel defibrillation mode which supports M defibrillation modules 102 to cooperatively perform a defibrillation operation. The method may further include: when N defibrillation modules 102 are detected, automatically determining a single-channel defibrillation mode, a dual-channel defibrillation mode, or an M-channel defibrillation mode as a target medical mode of the medical device; or selecting, based on a selection instruction of a user, one from the single-channel defibrillation mode, the dual-channel defibrillation mode, and the M-channel defibrillation mode as a target defibrillation mode of the medical device; and controlling the medical device to perform a defibrillation operation in the target defibrillation mode. Wherein M is greater than 2 and less than or equal to N, M and N are both integers.

For example, the method may further include detecting whether the medical device includes a monitoring module, wherein the monitoring module is configured to monitor physiological sign information of patient(s); and controlling the monitoring module to perform a monitoring operation in a monitoring mode, when the monitoring module is detected.

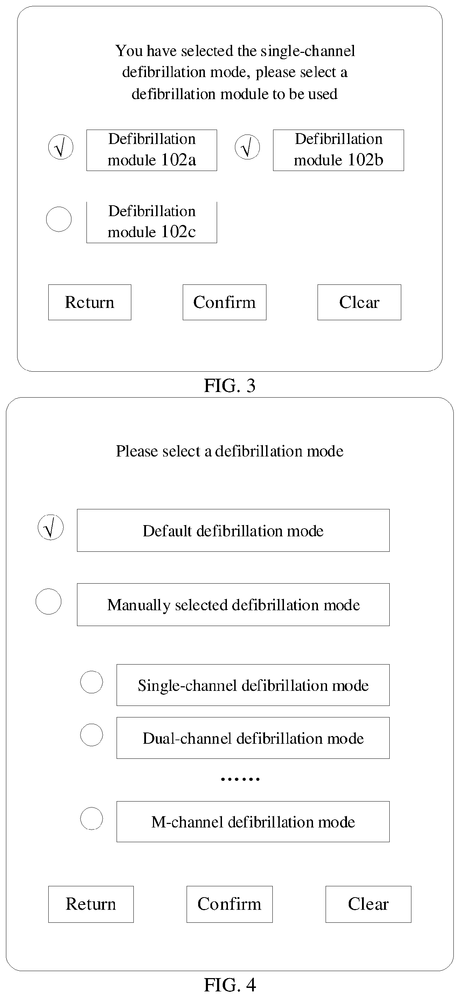

In some embodiments, the processor 101a may be configured to automatically or based on a selection instruction of a user, determine a defibrillation module 102 for performing a defibrillation operation in the target defibrillation mode before controlling the medical device to perform said defibrillation operation in the target defibrillation mode. As shown in FIG. 3, when the user has selected the single-channel defibrillation mode, the user can be indicated through an interactive interface to select a defibrillation module 102 (such as the defibrillation module 102a and the defibrillation module 102b) to perform a defibrillation operation in the single-channel defibrillation mode, and the user can click a confirmation button to issue a selection instruction for the processor 101a to control a selected defibrillation module 102a or defibrillation module 102b to perform the defibrillation operation.

In some embodiments, a default defibrillation mode may be preset in the host 101. As an example, a default defibrillation mode is preset in association with the number of defibrillation module(s) 102 detected. Specifically, when a single defibrillation module 102 is detected, the preset default defibrillation mode may be a single-channel defibrillation mode. When two defibrillation modules 102 are detected, the preset default defibrillation mode may be a dual-channel defibrillation mode. Thus, the preset default defibrillation mode is a defibrillation mode most frequently used by the user in an application scenario in which a corresponding number of defibrillation module(s) 102 is(are) combined in the medical device. Therefore, the user does not need to perform additional operations, and the processor 101a can automatically determine the default defibrillation mode as the target defibrillation mode of the medical device and perform a defibrillation operation that satisfies requirements. In the case of cardiac arrhythmia, there are strict requirements on the time consumption of defibrillation operation. Presetting the default defibrillation mode and automatically starting the default defibrillation mode can simplify the operation and reduce the time consumption.

In some embodiments, the processor 101a can be configured to, when a default defibrillation mode is preset in the host 101, first automatically determine the default defibrillation mode as the target defibrillation mode of the medical device, and then, upon receiving a selection instruction of a user, switch the target defibrillation mode to a defibrillation mode which is determined based on the selection instruction of the user. In this way, the user may be given the manual option to override the preset default defibrillation mode. Specifically, if the user accepts the default defibrillation mode, no additional selection is required, but if a different defibrillation mode is to be enabled, a manual selection can be made.

The medical device may further include a display to provide relevant indication to the user. In some embodiments, the processor 101a may be configured to control the display to display optional defibrillation mode(s) of the medical device. As shown in FIG. 4, an indication "Please select a defibrillation mode" may be displayed, and options of a default defibrillation mode and a manually selected defibrillation mode may be presented, wherein the default defibrillation mode is automatically selected. The user can select from a number of defibrillation mode options which are available. Based on the selection instruction of the user, the processor 101a may select a defibrillation mode from the optional defibrillation mode(s) as the target defibrillation mode.

In some embodiments, the processor 101a may automatically start the target defibrillation mode after determining the target defibrillation mode of the medical device, or may start the target defibrillation mode based on an operation instruction of a user. Then, the processor 101a may control the defibrillation module 102 corresponding to the target defibrillation mode to perform a defibrillation operation.

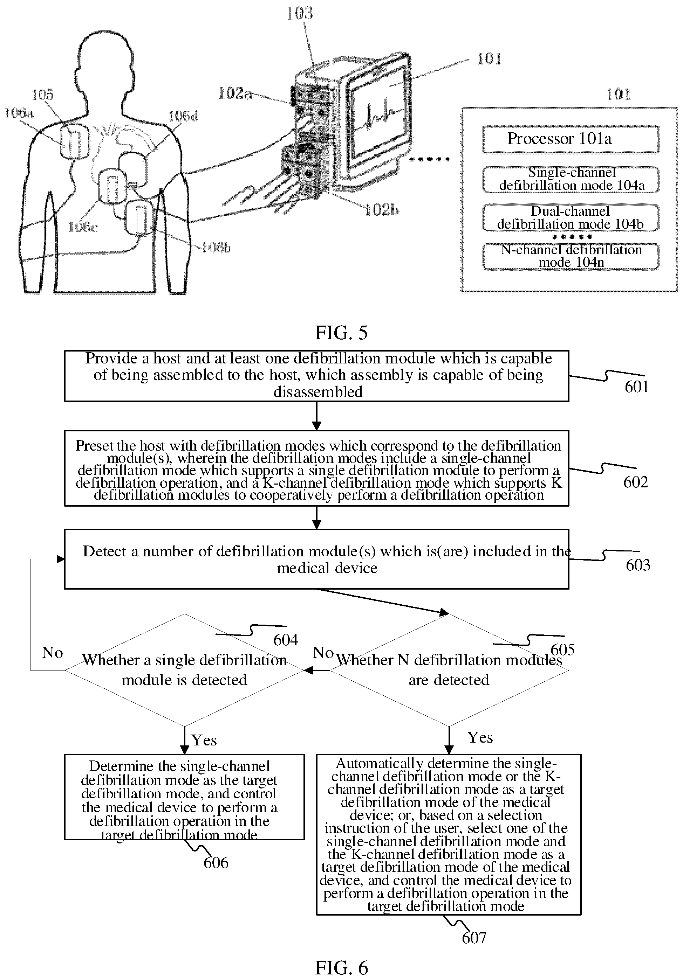

FIG. 5 is a diagram of a medical device with a defibrillation function according to an embodiment of this disclosure. As shown in FIG. 5, the medical device includes a host 101 having a processor 101 a, and at least one defibrillation module 102a and 102b (hereinafter collectively referred to as 102 if no distinction is made between the defibrillator modules) is capable of being assembled to the host, which assembly is capable of being disassembled, such that at least N defibrillation modules are capable of being combined to the medical device. N is an integer greater than or equal to 2. For example, three defibrillation modules 102a, 102b, and 102c (not shown) may be combined to the medical device.

The host 101 may be preset with defibrillation modes which correspond to the defibrillation module(s) 102, wherein the defibrillation modes include a single-channel defibrillation mode which supports a single defibrillation module 102 to perform a defibrillation operation, and a K-channel defibrillation mode which supports K defibrillation modules 102 to cooperatively perform a defibrillation operation, where K is an integer greater than 1, but less than or equal to N.

For example, when a maximum of N defibrillation modules 102 can be combined in the host 101, accordingly, a maximum of N defibrillation modes can be preset in the host 101, namely, a single-channel defibrillation mode 104a, a dual-channel defibrillation mode 104b, ... an N-channel defibrillation mode 104n, so as to give full play to the synergistic defibrillation effect of the combined defibrillation modules 102. When N is less than or equal to 2, K can be 2, the defibrillation modes can include a single-channel defibrillation mode and a dual-channel defibrillation mode. When N is greater than 2, K can be any number greater than 1, but less than or equal to N. It should be noted that when N is greater than 2, a dual-channel defibrillation mode may be preset in the host 101, and the K-channel defibrillation mode correspondingly includes a dual-channel defibrillation mode. For another example, when N is 3, K may be 1, 2 or 3, and accordingly, the defibrillation modes may include a single-channel defibrillation mode, a dual-channel defibrillation mode and a triple-channel defibrillation mode.

The processor 101a may be configured to detect a number of defibrillation module(s) 102 which is(are) included in the medical device. When a single defibrillation module 102 is detected, the processor 101a determines that the single-channel defibrillation mode is the target defibrillation mode, and controls the medical device to perform a defibrillation operation in the target defibrillation mode. When N defibrillation modules 102 are detected, the processor 101a automatically determines the single-channel defibrillation mode or the K-channel defibrillation mode as the target defibrillation mode of the medical device, or selects one from the single-channel defibrillation mode and the K-channel defibrillation mode as the target defibrillation mode of the medical device based on a selection instruction of a user, and controls the medical device to perform a defibrillation operation in the target defibrillation mode.

Various technical details of the medical device with a defibrillation function described in each embodiment of this disclosure can be combined into this embodiment individually or in combination, and are not repeated here.

FIG. 6 is a flowchart of a non-claimed method for configuring a medical device with a defibrillation function according to an embodiment of this disclosure. As shown in FIG. 6, the method includes, in step 601, provide a host and at least one defibrillation module which is capable of being assembled to the host, which assembly is capable of being disassembled. The method also includes, in step 602, preset the host with defibrillation modes which correspond to the defibrillation module(s), wherein the defibrillation modes include a single-channel defibrillation mode which supports a single defibrillation module to perform a defibrillation operation, and a K-channel defibrillation mode which supports K defibrillation modules to cooperatively perform a defibrillation operation, wherein K is an integer greater than 1. Please note that there is no particular limitation on the execution order of step 601 and step 602 as long as they do not affect their respective implementations. Steps 601 and 602 are the structure and setting configurations before an operation phase of the medical device with a defibrillation function. Furthermore, the medical device is configured for the operation phase, specifically including the following steps. In step 603, detect a number of defibrillation module(s) which is(are) included in the medical device. Then, different defibrillation modes are adapted according to the number of defibrillation module(s) detected. For example, in step 605 first determine whether N defibrillation modules are detected, wherein N is an integer equal to or greater than K, that is, equal to or greater than 2. When N defibrillation modules are detected (i.e., the determination result of step 605 is yes), automatically determine the single-channel defibrillation mode or the K-channel defibrillation mode as a target defibrillation mode of the medical device; or, based on a selection instruction of the user, select one of the single-channel defibrillation mode and the K-channel defibrillation mode as a target defibrillation mode of the medical device, and control the medical device to perform a defibrillation operation in the target defibrillation mode (step 607). If the determination result of step 605 is no, the process goes to step 604 to determine whether a single defibrillation module is detected. If yes, that is, when a single defibrillation module is detected, determine the single-channel defibrillation mode as the target defibrillation mode, and control the medical device to perform a defibrillation operation in the target defibrillation mode (step 606). If no defibrillator module is detected, the process returns to step 603 to continue detecting whether any defibrillator module is newly integrated into the medical device. In FIG. 6, similar to FIG. 2, firstly determine whether N defibrillator modules are detected, and then determine whether a single defibrillator module is detected, which can avoid redundant detection of a single defibrillator module, thereby improving process efficiency.

Various technical details of the medical device with a defibrillator function and the method for configuring a medical device with a defibrillator function described in each embodiment of this disclosure can be combined with this embodiment individually or in combination, and are not repeated here.

Now return to FIG. 5 to describe in detail the defibrillation operation in various defibrillation modes. As shown in FIG. 5, when using a dual-channel defibrillation mode (also known as a dual-channel sequential external defibrillation mode) or a multi-channel defibrillation mode (also known as a multi-channel sequential external defibrillation mode), electrodes are placed in pairs at different parts of human body to form several different discharge pathways through the heart. FIG. 5 shows an arrangement of two pairs of defibrillation electrodes when the dual-channel defibrillation mode is adopted, wherein one pair of defibrillation electrodes 106c and 106d are respectively placed on front and rear sides of the heart, while the other pair of defibrillation electrodes 106a and 106b are respectively placed on front and outer sides of the heart, thereby passing through different planes of the heart to form two independent discharge circuits. The purpose of defibrillation can be achieved by discharging the patient simultaneously or sequentially through these two discharge circuits. Note that, when the defibrillation electrodes are not distinguished from each other in the following text, each defibrillation electrode is uniformly represented by 106.

The processor 101a can be configured to: receive an operation for setting a defibrillation-related parameter (i.e., operation parameter), wherein the defibrillation-related parameter includes at least one of: an energy parameter of defibrillation (such as but not limited to energy, amplitude, and ratio of defibrillation waveform of multiple channels), a waveform parameter of defibrillation (time and electrical parameters which affects a waveform), and a time parameter of defibrillation (start time of discharge, end time of discharge for each defibrillation waveform, and a relationship between respective start time of discharge and/or respective end time of discharge for defibrillation waveform of different channels); and control the defibrillation module 102, which corresponds to the target defibrillation mode, so as to perform the defibrillation operation in the target defibrillation mode according to the set defibrillation-related parameter. An interaction interface for setting can be provided to the user to accept the operation of the user for setting the defibrillation-related parameter. In some embodiments, the defibrillation-related parameter may be preset (e.g., as default value) at the factory according to the customized requirements of the customer. In some embodiments, before starting the defibrillation operation in an actual application scenario, the user may be asked whether to manually adjust the defibrillation-related parameter. If no manual adjustment is made, the default defibrillation-related parameter is used. If a manual adjustment is made, the manually adjusted defibrillation-related parameter is used to perform the defibrillation operation. In this way, users are given the ability to adjust the defibrillation-related parameter to expected value based on their own experience according to specific defibrillation requirements. At the same time, users with relatively less experience can also use the medical device conveniently and ensure the effectiveness of defibrillation operation.

Specifically, when a medical device with a defibrillation function is combined with multiple defibrillation modules 102 (for example, a total of internally assembled defibrillation module(s) and externally inserted defibrillation module(s)), the processor 101a enables to display a dual-channel defibrillation interface, which interface can include guidance indication for placing dual sets of electrodes, so as to guide the user to correctly place the defibrillation electrodes 106a, 106b, 106c and 106d. Each defibrillation module 102a (or 102b) corresponds to its own defibrillation electrodes 106a and 106b (or 106c and 106d). When the target defibrillation mode, such as the dual-channel defibrillation mode, corresponds to at least two defibrillation modules 102a and 102b, the defibrillation electrodes 106a and 106b and the defibrillation electrodes 106c and 106d which correspond to each defibrillation module 102a and 102b, have different colors, so as to intuitively distinguish the groups of defibrillation electrodes 106a and 106b and of the defibrillation electrodes 106c and 106d.

In some embodiments, the processor 101a may enable the display to present various setting pop-up windows, such as but not limited to, a pop-up window for setting dual-channel defibrillation waveform(s), a pop-up window for delay setting dual-channel defibrillation waveform(s), etc., so as to indicate the user to set defibrillation-related parameters.

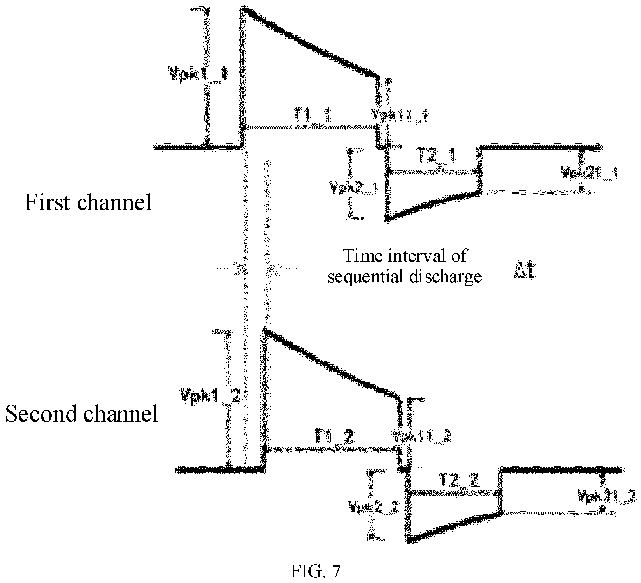

In some embodiments, the defibrillation waveform of the defibrillation module 102 includes a biphasic truncated waveform, as shown in FIG. 7. Taking a defibrillation waveform of a first channel of the defibrillation module 102a as an example, waveform parameters of defibrillation of the biphasic truncated waveform include a start voltage of first phase Vpk1_1, a truncated voltage of first phase Vpk11_1, a start voltage of second phase Vpk2_1, a truncated voltage of second phase Vpk21_1, exponential decay time for a voltage of first phase T1_1, and exponential decay time for a voltage of second phaseT2_1. Taking a defibrillation waveform of a second channel of the defibrillation module 102b as an example, waveform parameters of defibrillation of the biphasic truncated waveform include a start voltage of first phase Vpk1_2, a truncated voltage of first phase Vpk11_2, a start voltage of second phase Vpk2_2, a truncated voltage of second phase Vpk21_2, exponential decay time for a voltage of first phase T1_2, and exponential decay time for a voltage of second phaseT2_2. The user may manually set at least one of these waveform parameters of defibrillation via a setting operation. Phase voltage refers to a voltage between phase lines, i.e. a live wire and a neutral wire. The truncated voltage is a termination voltage, which refers to a lowest operation voltage at which the battery should no longer be discharged when the voltage drops during discharge. It should be noted that termination voltages are different for different battery types and different discharge conditions.

The defibrillation waveforms applied to the patient via different defibrillation modules 102 in different pathways may be the same or different. The medical device with a defibrillation function can control each defibrillation module 102 to output a defibrillation waveform of different shapes or a same shape but different amplitudes, by adjusting the waveform parameters of defibrillation for each defibrillation module 102 of each channel. Taking the example of a defibrillation waveform with two channels and each channel uses a biphasic truncated waveform as shown in FIG.7, the medical device with a defibrillator function can flexibly configure the above parameters to achieve two biphasic truncated waveforms with different shapes.

In some embodiments, when a single defibrillator module 102a performs the defibrillation operation, the time parameter of defibrillation includes a difference value between start time of discharge and end time of discharge for the single defibrillator module 102a, such as a time period between the start voltage of first phase Vpk1_1 and the truncated voltage of second phase Vpk21_1 as shown in FIG. 7. When at least two defibrillator modules 102a and 102b perform the defibrillation operation, the time parameter of defibrillation includes at least one of: a difference value between respective start time of discharge for any two defibrillator modules 102a and 102b (such as Δt as shown in FIG. 7), and a difference value between respective end time of discharge for any two defibrillator modules 102a and 102b, and a difference value between start time of discharge and end time of discharge for each defibrillator module 102a and 102b, for example, for the defibrillator module 102a, a time period between a start voltage of first phase Vpk1_1 and a truncated voltage of second phase Vpk21_1, and for the defibrillator module 102b, a time period between the start voltage of first phase Vpk1_2 and the truncated voltage of second phase Vpk21_2.

In some embodiments, the medical device with a defibrillation function may also include an ECG detection unit, which may be disposed inside the host 101, for example, as an ECG monitoring module which is inserted into or fixed to the host 101, or as a small unit which is connected to the defibrillation electrode 106 of the defibrillation module 102, such as an ECG detection unit 105 shown in FIG. 5. In some embodiments, each defibrillation electrode 106 may be attached with an ECG detection unit 105 to acquire an ECG signal from a corresponding part of a patient. The processor 101a may be configured to: obtain an ECG signal which is detected by the ECG detection unit 105; and control, based on the acquired ECG signal, start and stop of a defibrillation operation of the defibrillation module 102 in the target defibrillation mode, which module corresponds to the target defibrillation mode. For example, it is possible to analyze whether the target defibrillation mode is suitable for the patient based on the acquired ECG signal. For example, if the ECG signal indicates that the cardiac arrhythmia is caused by asystole, defibrillation should not be performed and should be stopped. For another example, if the ECG signal indicates that the patient has restored normal heart rhythm, the defibrillation operation should also be stopped.

A number of the ECG detection unit(s) 105 can be one or more, and each ECG detection unit 105 can detect one channel of ECG signal. When multiple ECG monitoring units 105 exist, the processor 101a can also be configured to: obtain multiple channels of ECG signal which are acquired by multiple ECG detection units 105; perform anti-interference process on the multiple channels of ECG signal, so as to obtain available ECG data; and control, based on the available ECG data, start and stop of the defibrillation operation of the defibrillation module 102 in the target defibrillation mode, which module corresponds to the target defibrillation mode. In this way, ECG data with less interference can be obtained as a reference for starting and stopping the defibrillation operation, thereby providing more reliable ECG data for starting and stopping the defibrillation operation and improving the effect of the defibrillation operation.

In some embodiments, the processor 101a may also be configured to: measure an impedance of a patient via the defibrillation electrode 106 before starting the target defibrillation mode; and adjust, according to the measured impedance of the patient, a charge voltage of the defibrillation module 102 which corresponds to the target defibrillation mode, so as to obtain an intensity of discharge current which intensity is within a preset range. Specifically, physical condition of the patient and even an environmental parameter (such as temperature, humidity, etc.) at the scene affects the impedance, so different charge voltages are required to obtain an intensity of discharge current which intensity is within a preset range. By measuring impedance of the patient through the defibrillation electrodes 106, the real-time impedance of the patient can be measured without introducing a new impedance measurement tool, and an appropriate charge voltage can be matched to ensure that the intensity of discharge current is always within a preset range and is not affected by a change of individual patient or environmental.

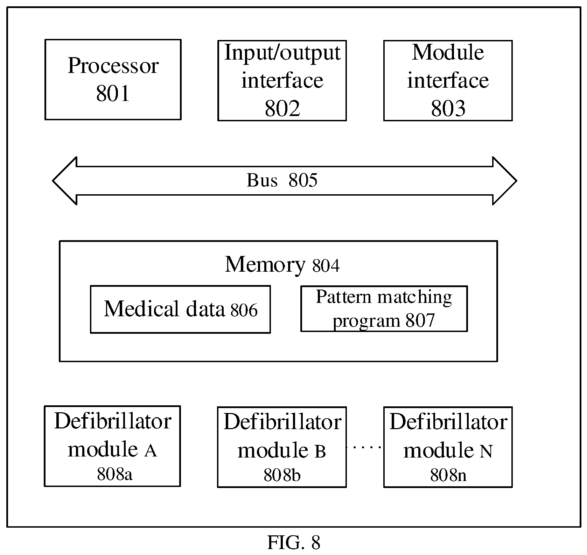

FIG. 8 is a structural block diagram of a medical device with a defibrillation function according to an embodiment of this disclosure. As shown in FIG. 8, the medical device may be a general data processing apparatus, including a general computer hardware structure. The medical device may include a processor 801, an input/output interface 802, a memory 804, and a module interface 803. The module interface 803 is constructed so as to conveniently combine a defibrillator module A 808a, a defibrillator module B 808b, ... , a defibrillator module N 808n, etc., to the medical device in a plug-in manner. In the following, if the defibrillator modules are not different, 808 is used to represent them uniformly. The hardware implementation of the processor 801 is similar to that of the processor 101a in FIG. 1, and is not described in detail here. The memory 804 is suitable for storing instructions or programs executable by the processor 801 and data generated when the instructions or programs are executed. For example, the memory 804 may store a pattern matching program 807 to match the combined defibrillator module A 808a, defibrillator module B 808b, ... defibrillator module N 808n with a suitable target defibrillator mode. The pattern matching program may follow a matching algorithm, for example, a single defibrillator module 808 matches a single-channel defibrillator mode, two defibrillator modules 808 match a single-channel defibrillator mode and a dual-channel defibrillator mode which are manually determined by the user, and so on. In some embodiments, when the pattern matching program 907 is executed, a method for configuring of a medical device with a defibrillation function according to various embodiments of this disclosure can be implemented. For example, the method for configuring may include the following steps. Preset the host with defibrillation modes which correspond to the defibrillation module(s), wherein the defibrillation modes include a single-channel defibrillation mode which supports a single defibrillation module to perform a defibrillation operation, and a dual-channel defibrillation mode which supports two defibrillation modules to cooperatively perform a defibrillation operation. Detect a number of defibrillation module(s) which is(are) included by the medical device. Determine the single-channel defibrillation mode as a target defibrillation mode of the medical device when a single defibrillation module is detected, and control the medical device to perform a defibrillation operation in said target defibrillation mode. Automatically determine the single-channel defibrillation mode or the dual-channel defibrillation mode, or select one from the single-channel defibrillation mode and the dual-channel defibrillation mode based on a selection instruction of a user, as a target defibrillation mode of the medical device, when two defibrillation modules are detected; and control the medical device to perform a defibrillation operation in said target defibrillation mode.

The matching algorithm may also consider other medical data 806 which are acquired from the medical device, such as electrocardiogram information, so as to match a target defibrillation mode (including not performing any defibrillation operation) which is suitable for the current condition of the heart of the patient.

Therefore, the processor 801 executes the commands and data stored in the memory 804, thereby executing the method flow of the embodiment of this disclosure as described above to realize the processing of data and the control of other devices. The bus 805 connects the above-mentioned multiple components together, and at the same time connects the above-mentioned components to a display controller and a display device, as well as an input/output interface 802. The input/output interface 802 can be interfaced to a mouse, keyboard, modem, network interface, touch input device, somatosensory input device, printer, and other devices known in the art to interact with the user, such as but not limited to for parameter setting, mode selection, module selection, defibrillation start/stop, etc.

The flowcharts and/or block diagrams of the methods, systems, and computer program products according to the embodiments of this disclosure describe various aspects of this disclosure. It is understood that each block of the flowchart illustrations and/or block diagrams, and combinations of blocks in the flowchart illustrations and/or block diagrams, can be implemented by computer program instructions. These computer program instructions may be provided to a processor of a general-purpose computer, a special-purpose computer or other programmable data processing device to produce a machine, so that the instructions (executed via the processor of the computer or other programmable data processing device) create means for implementing the functions/actions specified in the flowchart and/or block diagram blocks or blocks.

At the same time, as will be appreciated by those skilled in the art, various aspects of the embodiments of this disclosure may be implemented as a system, method or computer program product. Therefore, various aspects of the embodiments of this disclosure may take the following forms: a completely hardware implementation, a completely software implementation (including firmware, resident software, microcode, etc.), or an implementation that combines software aspects with hardware aspects, which may generally be referred to as a "circuit", "module" or "system" in this disclosure. Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (non-exhaustive list) of computer-readable storage media would include the following: an electrical connection having one or more wires, a portable computer floppy disk, a hard disk, a random access memory (RAM), a read-only memory (ROM), an erasable programmable read-only memory (EPROM or flash memory), optical fiber, a portable compact disk read-only memory (CD-ROM), an optical storage device, a magnetic storage device, or any suitable combination of the foregoing. In the context of embodiments of this disclosure, a computer-readable storage medium may be any tangible medium that can contain or store a program for use by or in conjunction with an instruction execution system, apparatus, or device.

A computer readable signal medium may include a propagated data signal with computer readable program code embodied therein, such as in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in conjunction with an instruction execution system, apparatus, or device.

Program code embodied on a computer readable medium may be transmitted using any appropriate medium including, but not limited to, wireless, wired, optical fiber cable, RF, etc., or any suitable combination of the foregoing.

Computer program code for performing operations for various aspects of this disclosure can be written in any combination of one or more programming languages, including: object-oriented programming languages such as Java, Smalltalk, C++, PHP, Python, etc.; and conventional procedural programming languages such as the "C" programming language or similar programming languages. The program code may execute entirely on a computer of the user, partly on a computer of the user, partly on a computer of the user and partly on a remote computer, or entirely on the remote computer or server, as a stand-alone software package. In the latter case, the remote computer may be connected to a computer of the user through any type of network, including a local area network (LAN) or a wide area network (WAN), or may be connected to an external computer (e.g., through the Internet using an Internet service provider).

These computer program instructions may also be stored in a computer-readable medium that can direct a computer, other programmable data processing device, or other apparatus to operate in a particular manner, so that the instructions stored in the computer-readable medium produce an article of manufacture including instructions for implementing the functions/actions specified in the flowchart and/or block diagram blocks or blocks.

The computer program instructions may also be loaded onto a computer, other programmable data processing device or other apparatus so that a series of operable steps are performed on the computer, other programmable device or other apparatus to produce a computer-implemented process, so that the instructions executed on the computer or other programmable device provide a process for implementing the functions/actions specified in the flowchart and/or block diagram block or blocks.

In addition, although exemplary embodiments have been described herein, the scope thereof includes any and all embodiments based on this disclosure having equivalent elements, modifications, omissions, combinations (e.g., schemes that cross various embodiments), adaptations, or changes.

Accordingly, it is intended that the specification and examples be considered as exemplary only, with a true scope being indicated by the following claims.

The above description is intended to be illustrative rather than limiting. For example, the above-described examples (or one or more aspects thereof) may be used in combination with each other. Other embodiments may come such skilled in the art upon reading the above description. Additionally, in the above detailed description, various features may be grouped together to simplify the disclosure. This should not be interpreted as intending that an unclaimed disclosed feature is essential to any claim.

The scope of this disclosure should be determined with reference to the appended claims.

## Claims

1. A medical device with a defibrillation function comprising:
a host (101), which is capable of being assembled with at least one defibrillation module (102, 102a, 102b), which assembly is capable of being disassembled, such that at least two defibrillation modules (102, 102a, 102b) are capable of being combined to the medical device;
the host (101) is preset with a single-channel defibrillation mode (104a) which supports a single defibrillation module to perform a defibrillation operation, and a dual-channel defibrillation mode (104b) which supports two defibrillation modules to cooperatively perform a defibrillation operation;
wherein the host (101) comprises a processor (101a), which is configured to:
detect a number of defibrillation module(s) (102) which is(are) included by the medical device;
automatically determine the single-channel defibrillation mode (104a) or the dual-channel defibrillation mode (104b), or select one from the single-channel defibrillation mode (104a) and the dual-channel defibrillation mode (104b) based on a selection instruction of a user, as a target defibrillation mode of the medical device, when two defibrillation modules (102) are detected; and control the medical device to perform a defibrillation operation in said target defibrillation mode.

2. The medical device with a defibrillation function according to claim 1, **characterized in that**, the processor (101a) is further configured to:
determine the single-channel defibrillation mode (104a) as a target defibrillation mode of the medical device when a single defibrillation module (102) is detected, and control the medical device to perform a defibrillation operation in said target defibrillation mode.

3. The medical device with a defibrillation function according to claim 2, **characterized in that**, a preset defibrillation mode further comprises an M-channel defibrillation mode (104m) which supports M defibrillation modules to cooperatively perform a defibrillation operation;
the processor (101a) is further configured to:
automatically determine the single-channel defibrillation mode (104a), the dual-channel defibrillation mode (104b) or the M-channel defibrillation mode (104m), or select one from the single-channel defibrillation mode (104a), the dual-channel defibrillation mode (104b) and the M-channel defibrillation mode (104m) based on a selection instruction of a user, as a target defibrillation mode of the medical device; when N defibrillation modules (102) are detected; and
control the medical device to perform a defibrillation operation in said target defibrillation mode;
wherein M is greater than 2 and less than or equal to N, M and N are both integers.

4. The medical device with a defibrillation function according to any one of claims 2-3, **characterized in that**, the processor (101a) is further configured to:
determine, automatically or based on a selection instruction of a user, a defibrillation module (102) for performing the defibrillation operation in the target defibrillation mode, before controlling the medical device to perform the defibrillation operation in the target defibrillation mode.

5. The medical device with a defibrillation function according to any one of claims 2-4, **characterized in that**, the processor (101a) is further configured to:
automatically determine a default defibrillation mode as the target defibrillation mode of the medical device, when the default defibrillation mode is preset in the host (101); or
automatically determine a default defibrillation mode as the target defibrillation mode of the medical device at first, when the default defibrillation mode is preset in the host (101); and then, in response to a selection instruction of a user, switch the target defibrillation mode to a defibrillation mode which is determined based on said selection instruction of said user.

6. The medical device with a defibrillation function according to any one of claims 2-5, **characterized in that**, further comprising a display; wherein the processor (101a) is further configured to:
control the display to display optional defibrillation mode(s) of the medical device; and
select, based on a selection instruction of a user, a defibrillation mode from the optional defibrillation mode(s), as the target defibrillation mode.

7. The medical device with a defibrillation function according to any one of claims 2-6, **characterized in that**, the processor (101a) is further configured to:
start, automatically or based on an operation instruction of a user, the target defibrillation mode, after determining/selecting the target defibrillation mode of the medical device.

8. The medical device with a defibrillation function according to any one of claims 2-7, **characterized in that**, the host (101) is provided with at least one accommodation cavity (103), and the defibrillator module (102) is capable of being accommodated inside the accommodation cavity (103) in a plug-in manner.

9. The medical device with a defibrillation function according to any one of claims 2-8, **characterized in that**, when the medical device further comprises a monitoring module, the processor (101a) is further configured to control the monitoring module to perform a monitoring operation in a monitoring mode, so as to monitor physiological sign information of patient(s).

10. The medical device with a defibrillation function according to claim 9, **characterized in that**, the monitoring module is capable of being assembled at the host (101), which assembly is capable of being disassembled; or
the physiological sign information at least comprises an electrocardiogram signal; the processor is further configured to: control start and stop of a defibrillation operation of corresponding defibrillation module(s) in the target defibrillation mode, according to the electrocardiogram signal which is detected by the monitoring module.

11. The medical device with a defibrillation function according to any one of claims 2-10, **characterized in that**, the processor (101a) is further configured to:
receive an operation for setting a defibrillation-related parameter, wherein the defibrillation-related parameter comprises at least one of: an energy parameter of defibrillation, a waveform parameter of defibrillation, and a time parameter of defibrillation; and
control defibrillation module(s), which correspond(s) to the target defibrillation mode, to perform a defibrillation operation in the target defibrillation mode according to the set defibrillation-related parameter.

12. The medical device with a defibrillation function according to claim 11, **characterized in that**,
when a single defibrillation module (102a) performs the defibrillation operation, the time parameter of defibrillation comprises a difference value between start time of discharge and end time of discharge for said single defibrillation module (102a);
when at least two defibrillation modules (102a, 102b) perform the defibrillation operation, the time parameter of defibrillation comprises at least one of: a difference value between respective start time of discharge for any two defibrillation modules (102a, 102b), a difference value between respective end time of discharge for any two defibrillation modules (102a, 102b), and a difference value between start time of discharge and end time of discharge for each defibrillation module (102); or
a defibrillation waveform of the defibrillation module (102) is a biphasic truncated waveform; the waveform parameter of defibrillation comprises at least one of: a start voltage of first phase, a truncated voltage of first phase, a start voltage of second phase, a truncated voltage of second phase, exponential decay time for a voltage of first phase, and exponential decay time for a voltage of second phase.

13. The medical device with a defibrillation function according to any one of claims 2-9, **characterized in that**, further comprising an electrocardiogram detection unit (105), wherein the electrocardiogram detection unit (105) is arranged inside the host (101) or connected with a defibrillation electrode (106) of a defibrillation module (102);
the processor (101a) is further configured to:
acquire an electrocardiogram signal which is detected by the electrocardiogram detection unit (105); and
control start and stop of a defibrillation operation of defibrillation module(s) (102) in the target defibrillation mode, which module(s) correspond(s) to the target defibrillation mode, based on the acquired electrocardiogram signal.

14. The medical device with a defibrillation function according to claim 13, **characterized in that**, a number of electrocardiogram detection unit(s) is one or more, and each electrocardiogram detection unit (105) is capable of detecting one channel of electrocardiogram signal;
when multiple electrocardiogram detection units (105) exist, the processor (101a) is further configured to:
acquire multiple channels of electrocardiogram signal which are acquired by the electrocardiogram detection units (105);
perform anti-interference process on the multiple channels of electrocardiogram signal, so as to obtain available electrocardiogram data; and
control start and stop of the defibrillation operation of the defibrillation module(s) (102) in the target defibrillation mode, which module(s) correspond(s) to the target defibrillation mode, based on the available electrocardiogram data.

15. The medical device with a defibrillation function according to any one of claims 2-14, **characterized in that**, the processor (101a) is further configured to:
measure impedance of a patient by a defibrillation electrode (106) before starting the target defibrillation mode; and
adjust, according to the measured impedance of the patient, a charge voltage of defibrillation module(s) (102) which correspond(s) to the target defibrillation mode, so as to obtain an intensity of discharge current which intensity is within a preset range;
preferably, each defibrillation module (102) correspondingly has its own defibrillation electrode (106a, 106b, 106c, 106d); when the target defibrillation mode corresponds to at least two defibrillation modules (102a, 102b), defibrillation electrodes (106a, 106b, 106c, 106d) which correspond to different defibrillation modules have different colors.

## Patentansprüche

1. Eine medizinische Vorrichtung mit Defibrillationsfunktion, umfassend:
einen Host (101), der mit mindestens einem Defibrillationsmodul (102, 102a, 102b) verbunden werden kann, wobei diese Baugruppe zerlegt werden kann, sodass mindestens zwei Defibrillationsmodule (102, 102a, 102b) zu der medizinischen Vorrichtung kombiniert werden können;
der Host (101) ist mit einem Ein-Kanal-Defibrillationsmodus (104a) voreingestellt, der ein einzelnes Defibrillationsmodul zur Durchführung eines Defibrillationsvorgangs unterstützt, sowie mit einem Zwei-Kanal-Defibrillationsmodus (104b), der zwei Defibrillationsmodule zur gemeinsamen Durchführung eines Defibrillationsvorgangs unterstützt;
wobei der Host (101) einen Prozessor (101a) umfasst, der so ausgelegt ist, dass er:
eine Anzahl von Defibrillationsmodulen (102) erfasst, die in der medizinischen Vorrichtung enthalten sind;
automatisch den Ein-Kanal-Defibrillationsmodus (104a) oder den Zwei-Kanal-Defibrillationsmodus (104b) festlegt, oder auf der Grundlage einer Auswahlanweisung eines Benutzers den Ein-Kanal-Defibrillationsmodus (104a) oder den Zwei-Kanal-Defibrillationsmodus (104b) als Ziel-Defibrillationsmodus der medizinischen Vorrichtung auswählt, wenn zwei Defibrillationsmodule (102) erkannt werden; und die medizinische Vorrichtung so steuert, dass sie einen Defibrillationsvorgang in dem genannten Ziel-Defibrillationsmodus durchführt.

2. Die medizinische Vorrichtung mit Defibrillationsfunktion nach Anspruch 1, **dadurch gekennzeichnet, dass** der Prozessor (101a) ferner so ausgelegt ist, dass er:
den Ein-Kanal-Defibrillationsmodus (104a) als Ziel-Defibrillationsmodus der medizinischen Vorrichtung bestimmt, wenn ein einzelnes Defibrillationsmodul (102) erkannt wird, und die medizinische Vorrichtung so steuert, dass sie einen Defibrillationsvorgang in dem genannten Ziel-Defibrillationsmodus durchführt.

3. Die medizinische Vorrichtung mit Defibrillationsfunktion nach Anspruch 2, **dadurch gekennzeichnet, dass** ein voreingestellter Defibrillationsmodus ferner einen M-Kanal-Defibrillationsmodus (104m) umfasst, der M Defibrillationsmodule unterstützt, um gemeinsam einen Defibrillationsvorgang durchzuführen;
der Prozessor (101a) ferner so konfiguriert ist, dass er:
automatisch den Ein-Kanal-Defibrillationsmodus (104a), den Zwei-Kanal-Defibrillationsmodus (104b) oder den M-Kanal-Defibrillationsmodus (104m) bestimmt oder den Ein-Kanal-Defibrillationsmodus (104a), den Zweikanal-Defibrillationsmodus (104b) oder den M-Kanal-Defibrillationsmodus (104m) auf der Grundlage einer Auswahlanweisung eines Benutzers als Ziel-Defibrillationsmodus der medizinischen Vorrichtung auswählt, wenn N Defibrillationsmodule (102) erkannt werden; und
die medizinische Vorrichtung so steuert, dass sie einen Defibrillationsvorgang in dem genannten Ziel-Defibrillationsmodus durchführt;
wobei M größer als 2 und kleiner oder gleich N ist und wobei M und N beide ganze Zahlen sind.

4. Die medizinische Vorrichtung mit Defibrillationsfunktion nach einem der Ansprüche 2 bis 3, **dadurch gekennzeichnet, dass** der Prozessor (101a) ferner so ausgelegt ist, dass er:
automatisch oder auf der Grundlage einer Auswahlanweisung eines Benutzers ein Defibrillationsmodul (102) zur Durchführung des Defibrillationsvorgangs im Ziel-Defibrillationsmodus bestimmt, bevor er die medizinische Vorrichtung zur Durchführung des Defibrillationsvorgangs im Ziel-Defibrillationsmodus steuert.

5. Die medizinische Vorrichtung mit Defibrillationsfunktion nach einem der Ansprüche 2 bis 4, **dadurch gekennzeichnet, dass** der Prozessor (101a) ferner so ausgelegt ist, dass er:
automatisch einen Standard-Defibrillationsmodus als Ziel-Defibrillationsmodus der medizinischen Vorrichtung bestimmt, wenn der Standard-Defibrillationsmodus im Host (101) voreingestellt ist; oder
zunächst automatisch einen Standard-Defibrillationsmodus als Ziel-Defibrillationsmodus der medizinischen Vorrichtung bestimmt, wenn der Standard-Defibrillationsmodus im Host (101) voreingestellt ist, und anschließend als Reaktion auf eine Auswahlanweisung eines Benutzers den Ziel-Defibrillationsmodus auf einen Defibrillationsmodus umschaltet, der auf der Grundlage der genannten Auswahlanweisung des genannten Benutzers bestimmt wird.

6. Die medizinische Vorrichtung mit Defibrillationsfunktion nach einem der Ansprüche 2 bis 5, **dadurch gekennzeichnet, dass** sie ferner eine Anzeige umfasst; wobei der Prozessor (101a) ferner so ausgelegt ist, dass er:
die Anzeige so steuert, dass optionale Defibrillationsmodi der medizinischen Vorrichtung angezeigt werden; und
auf der Grundlage einer Auswahlanweisung eines Benutzers einen Defibrillationsmodus aus den optionalen Defibrillationsmodi als Ziel-Defibrillationsmodus auswählt.

7. Die medizinische Vorrichtung mit Defibrillationsfunktion nach einem der Ansprüche 2 bis 6, **dadurch gekennzeichnet, dass** der Prozessor (101a) ferner so ausgelegt ist, dass er:
den Ziel-Defibrillationsmodus automatisch oder auf der Grundlage einer Bedienungsanweisung eines Benutzers startet, nachdem der Ziel-Defibrillationsmodus der medizinischen Vorrichtung bestimmt/ausgewählt wurde.

8. Die medizinische Vorrichtung mit Defibrillationsfunktion nach einem der Ansprüche 2 bis 7, **dadurch gekennzeichnet, dass** der Host (101) mit mindestens einem Aufnahmeraum (103) versehen ist und das Defibrillationsmodul (102) in den Aufnahmeraum (103) einsteckbar aufgenommen werden kann.

9. Die medizinische Vorrichtung mit Defibrillationsfunktion nach einem der Ansprüche 2 bis 8, **dadurch gekennzeichnet, dass**, wenn die medizinische Vorrichtung ferner ein Überwachungsmodul umfasst, der Prozessor (101a) ferner so ausgelegt ist, dass er das Überwachungsmodul so steuert, dass es einen Überwachungsvorgang in einem Überwachungsmodus durchführt, um physiologische Signaldaten eines oder mehrerer Patienten zu überwachen.

10. Die medizinische Vorrichtung mit Defibrillationsfunktion nach Anspruch 9, **dadurch gekennzeichnet, dass** das Überwachungsmodul an dem Host (101) montiert werden kann, wobei diese Anbringung wieder rückgängig gemacht werden kann; oder
die physiologischen Signaldaten zumindest ein Elektrokardiogrammsignal umfassen; der Prozessor ferner so ausgelegt ist, dass er: den Start und den Stopp eines Defibrillationsvorgangs des/der entsprechenden Defibrillationsmoduls/-module im Ziel-Defibrillationsmodus entsprechend dem vom Überwachungsmodul erfassten Elektrokardiogrammsignal steuert.

11. Die medizinische Vorrichtung mit Defibrillationsfunktion nach einem der Ansprüche 2 bis 10, **dadurch gekennzeichnet, dass** der Prozessor (101a) ferner so ausgelegt ist, dass er:
einen Befehl zum Einstellen eines defibrillationsbezogenen Parameters empfängt, wobei der defibrillationsbezogene Parameter mindestens einen der folgenden umfasst: einen Energieparameter der Defibrillation, einen Wellenformparameter der Defibrillation oder einen Zeitparameter der Defibrillation; und
ein oder mehrere Defibrillationsmodule, die dem Ziel-Defibrillationsmodus entsprechen, so steuert, dass sie einen Defibrillationsvorgang im Ziel-Defibrillationsmodus gemäß dem eingestellten defibrillationsbezogenen Parameter durchführen.

12. Die medizinische Vorrichtung mit Defibrillationsfunktion nach Anspruch 11, **dadurch gekennzeichnet, dass**,
wenn ein einzelnes Defibrillationsmodul (102a) den Defibrillationsvorgang durchführt, der Zeitparameter der Defibrillation einen Differenzwert zwischen der Startzeit der Entladung und der Endzeit der Entladung für das einzelne Defibrillationsmodul (102a) umfasst;
wenn mindestens zwei Defibrillationsmodule (102a, 102b) den Defibrillationsvorgang durchführen, der Zeitparameter der Defibrillation mindestens eines der folgenden Elemente umfasst: einen Differenzwert zwischen den jeweiligen Entladungsstartzeiten zweier beliebiger Defibrillationsmodule (102a, 102b), einen Differenzwert zwischen den jeweiligen Entladungsendzeiten zweier beliebiger Defibrillationsmodule (102a, 102b) sowie einen Differenzwert zwischen dem Startzeitpunkt der Entladung und dem Endzeitpunkt der Entladung für jedes Defibrillationsmodul (102); oder
wenn eine Defibrillationswellenform des Defibrillationsmoduls (102) eine biphasische, abgeschnittene Wellenform ist; der Wellenformparameter der Defibrillation mindestens einen der folgenden Werte umfasst: eine Startspannung der ersten Phase,
eine abgeschnittene Spannung der ersten Phase, eine Startspannung der zweiten Phase, eine abgeschnittene Spannung der zweiten Phase, eine exponentielle Abklingzeit für eine Spannung der ersten Phase und eine exponentielle Abklingzeit für eine Spannung der zweiten Phase.

13. Die medizinische Vorrichtung mit Defibrillationsfunktion nach einem der Ansprüche 2 bis 9, **dadurch gekennzeichnet, dass** sie ferner eine Elektrokardiogramm-Erfassungseinheit (105) umfasst, wobei die Elektrokardiogramm-Erfassungseinheit (105) innerhalb des Hosts (101) angeordnet oder mit einer Defibrillationselektrode (106) eines Defibrillationsmoduls (102) verbunden ist;
der Prozessor (101a) ferner so ausgelegt ist, dass er:
ein von der Elektrokardiogramm-Erfassungseinheit (105) erfasstes Elektrokardiogrammsignal erfasst; und
den Start und Stopp eines Defibrillationsvorgangs eines oder mehrerer Defibrillationsmodule (102) im Ziel-Defibrillationsmodus steuert, wobei dieses oder diese Module dem Ziel-Defibrillationsmodus entsprechen, auf der Grundlage des erfassten Elektrokardiogrammsignals.

14. Die medizinische Vorrichtung mit Defibrillationsfunktion nach Anspruch 13, **dadurch gekennzeichnet, dass** die Anzahl der Elektrokardiogramm-Erfassungseinheiten eins oder mehr beträgt und jede Elektrokardiogramm-Erfassungseinheit (105) in der Lage ist, einen Kanal des Elektrokardiogrammsignals zu erfassen;
wenn mehrere Elektrokardiogramm-Erfassungseinheiten (105) vorhanden sind, ist der Prozessor (101a) ferner so ausgelegt, dass er:
mehrere Kanäle des Elektrokardiogrammsignals erfasst, die von den Elektrokardiogramm-Erfassungseinheiten (105) erfasst werden;
einen Entstörungsprozess an den mehreren Kanälen des Elektrokardiogrammsignals durchführt, um verfügbare Elektrokardiogrammdaten zu erhalten; und
Start und Stopp des Defibrillationsvorgangs des oder der Defibrillationsmodule (102) im Ziel-Defibrillationsmodus steuert, wobei das oder die Module dem Ziel-Defibrillationsmodus entsprechen, basierend auf den verfügbaren Elektrokardiogrammdaten.

15. Die medizinische Vorrichtung mit Defibrillationsfunktion nach einem der Ansprüche 2 bis 14, **dadurch gekennzeichnet, dass** der Prozessor (101a) ferner so ausgelegt ist, dass er:
die Impedanz eines Patienten mittels einer Defibrillationselektrode (106) vor dem Start des Ziel-Defibrillationsmodus misst; und
die Ladespannung des oder der Defibrillationsmodule (102), die dem angestrebten Defibrillationsmodus entsprechen, entsprechend der gemessenen Impedanz des Patienten so anpasst, dass eine Entladestromstärke erzielt wird, die innerhalb eines voreingestellten Bereichs liegt;
vorzugsweise jedes Defibrillationsmodul (102) entsprechend über eine eigene Defibrillationselektrode (106a, 106b, 106c, 106d) verfügt; wenn der Ziel-Defibrillationsmodus mindestens zwei Defibrillationsmodulen (102a, 102b) entspricht, Defibrillationselektroden (106a, 106b, 106c, 106d), die verschiedenen Defibrillationsmodulen entsprechen, unterschiedliche Farben aufweisen.

## Revendications

1. Dispositif médical avec fonction de défibrillation comprenant :
un hôte (101), lequel peut être assemblé avec au moins un module de défibrillation (102, 102a, 102b), lequel assemblage peut être désassemblé, de telle sorte qu'au moins deux modules de défibrillation (102, 102a, 102b) peuvent être combinés sur le dispositif médical ;
l'hôte (101) est préréglé avec un mode de défibrillation à unique canal (104a) lequel prend en charge un unique module de défibrillation pour réaliser une opération de défibrillation, et un mode de défibrillation à deux canaux (104b) lequel prend en charge deux modules de défibrillation pour réaliser conjointement une opération de défibrillation ;
où l'hôte (101) comprend un processeur (101a), lequel est configuré pour :
détecter un nombre de module(s) de défibrillation (102) le(s)quel(s) est (sont) inclus par le dispositif médical ;
déterminer automatiquement le mode de défibrillation à unique canal (104a) ou le mode de défibrillation à deux canaux (104b), ou sélectionner un mode parmi le mode de défibrillation à unique canal (104a) et le mode de défibrillation à deux canaux (104b) sur la base d'une instruction de sélection d'un utilisateur, en tant que mode de défibrillation cible du dispositif médical, lorsque deux modules de défibrillation (102) sont détectés ; et commander le dispositif médical pour réaliser une opération de défibrillation dans ledit mode de défibrillation cible.

2. Dispositif médical avec fonction de défibrillation selon la revendication 1, **caractérisé en ce que** le processeur (101a) est en outre configuré pour :
déterminer le mode de défibrillation à unique canal (104a) en tant que mode de défibrillation cible du dispositif médical lorsqu'un unique module de défibrillation (102) est détecté, et commander le dispositif médical pour réaliser une opération de défibrillation dans ledit mode de défibrillation cible.

3. Dispositif médical avec fonction de défibrillation selon la revendication 2, **caractérisé en ce qu'**un mode de défibrillation préréglé comprend en outre un mode de défibrillation à M canaux (104m) lequel prend en charge M modules de défibrillation pour réaliser conjointement une opération de défibrillation ;
le processeur (101a) est en outre configuré pour :
déterminer automatiquement le mode de défibrillation à unique canal (104a), le mode de défibrillation à deux canaux (104b) ou le mode de défibrillation à M canaux (104m), ou sélectionner un mode parmi le mode de défibrillation à unique canal (104a), le mode de défibrillation à deux canaux (104b) et le mode de défibrillation à M canaux (104m) sur la base d'une instruction de sélection d'un utilisateur, en tant que mode de défibrillation cible du dispositif médical, lorsque N modules de défibrillation (102) sont détectés ; et
commander le dispositif médical pour réaliser une opération de défibrillation dans ledit mode de défibrillation cible ;
où M est supérieur à 2 et inférieur ou égal à N, M et N sont tous deux des entiers.

4. Dispositif médical avec fonction de défibrillation selon l'une quelconque des revendications 2 et 3, **caractérisé en ce que** le processeur (101a) est en outre configuré pour :
déterminer, automatiquement ou sur la base d'une instruction de sélection d'un utilisateur, un module de défibrillation (102) en vue de réaliser une opération de défibrillation dans le mode de défibrillation cible, avant de commander le dispositif médical pour réaliser l'opération de défibrillation dans le mode de défibrillation cible.

5. Dispositif médical avec fonction de défibrillation selon l'une quelconque des revendications 2 à 4, **caractérisé en ce que** le processeur (101a) est en outre configuré pour :
déterminer automatiquement un mode de défibrillation par défaut en tant que mode de défibrillation cible du dispositif médical, lorsque le mode de défibrillation par défaut est préréglé dans l'hôte (101) ; ou
déterminer automatiquement un mode de défibrillation par défaut en tant que mode de défibrillation cible du dispositif médical en premier lieu, lorsque le mode de défibrillation par défaut est préréglé dans l'hôte (101) ; et ensuite, en réponse à une instruction de sélection d'un utilisateur, passer le mode de défibrillation cible à un certain mode de défibrillation lequel est déterminé sur la base de ladite instruction de sélection dudit utilisateur.

6. Dispositif médical avec fonction de défibrillation selon l'une quelconque des revendications 2 à 5, **caractérisé en ce que**, comprenant en outre un affichage ; où le processeur (101a) est en outre configuré pour :
commander l'affichage pour afficher un (des) mode(s) de défibrillation optionnel(s) du dispositif médical ; et
sélectionner, sur la base d'une instruction de sélection d'un utilisateur, un mode de défibrillation parmi le(s) mode(s) de défibrillation optionnel(s), en tant que mode de défibrillation cible.

7. Dispositif médical avec fonction de défibrillation selon l'une quelconque des revendications 2 à 6, **caractérisé en ce que** le processeur (101a) est en outre configuré pour :
démarrer, automatiquement ou sur la base d'une instruction de mise en œuvre d'un utilisateur, le mode de défibrillation cible, après avoir déterminé/sélectionné le mode de défibrillation cible du dispositif médical.

8. Dispositif médical avec fonction de défibrillation selon l'une quelconque des revendications 2 à 7, **caractérisé en ce que** l'hôte (101) est doté d'au moins une cavité de réception (103), et le module de défibrillation (102) peut être reçu à l'intérieur de la cavité de réception (103) d'une manière enfichable.

9. Dispositif médical avec fonction de défibrillation selon l'une quelconque des revendications 2 à 8, **caractérisé en ce que**, lorsque le dispositif médical comprend en outre un module de surveillance, le processeur (101a) est en outre configuré pour commander le module de surveillance pour réaliser une opération de surveillance dans un mode de surveillance, de sorte à surveiller une information de signe physiologique de patient(s).

10. Dispositif médical avec fonction de défibrillation selon la revendication 9, **caractérisé en ce que** le module de surveillance peut être assemblé au niveau de l'hôte (101), lequel assemblage peut être désassemblé ; ou
l'information de signe physiologique comprend au moins un signal d'électrocardiogramme ; le processeur est en outre configuré pour : commander un départ et un arrêt d'une opération de défibrillation de module(s) de défibrillation correspondant(s) dans le mode de défibrillation cible, en fonction du signal d'électrocardiogramme lequel est détecté par le module de surveillance.

11. Dispositif médical avec fonction de défibrillation selon l'une quelconque des revendications 2 à 10, **caractérisé en ce que** le processeur (101a) est en outre configuré pour :
recevoir une opération de réglage d'un paramètre relatif à une défibrillation, où le paramètre relatif à une défibrillation comprend au moins un paramètre parmi : un paramètre d'énergie de défibrillation, un paramètre de forme d'onde de défibrillation, et un paramètre de durée de défibrillation ; et
commander un (des) module(s) de défibrillation, le(s)quel(s) correspond(ent) au mode de défibrillation cible, pour réaliser une opération de défibrillation dans le mode de défibrillation cible en fonction du paramètre relatif à une défibrillation réglé.

12. Dispositif médical avec fonction de défibrillation selon la revendication 11, **caractérisé en ce que**
lorsqu'un unique module de défibrillation (102a) réalise l'opération de défibrillation, le paramètre de durée de défibrillation comprend une valeur de différence entre un instant de début de décharge et un instant de fin de décharge pour ledit unique module de défibrillation (102a) ;
lorsque au moins deux modules de défibrillation (102a, 102b) réalisent l'opération de défibrillation, le paramètre de durée de défibrillation comprend au moins une valeur parmi : une valeur de différence entre des instants de début de décharge respectifs pour les deux modules de défibrillation (102a, 102b), une valeur de différence entre des instants de fin de décharge respectifs pour les deux modules de défibrillation (102a, 102b), et une valeur de différence entre un instant de début de décharge et un instant de fin de décharge pour chaque module de défibrillation (102) ; ou
une forme d'onde de défibrillation du module de défibrillation (102) est une forme d'onde tronquée biphasique ; le paramètre de forme d'onde de défibrillation comprend au moins un élément parmi : une tension de début de première phase, une tension tronquée de première phase, une tension de début de seconde phase, une tension tronquée de seconde phase, une durée de décroissance exponentielle d'une tension de première phase, et une durée de décroissance exponentielle d'une tension de seconde phase.

13. Dispositif médical avec fonction de défibrillation selon l'une quelconque des revendications 2 à 9, **caractérisé en ce que**, comprenant en outre une unité de détection d'électrocardiogramme (105), où l'unité de détection d'électrocardiogramme (105) est agencée à l'intérieur de l'hôte (101) ou reliée à une électrode de défibrillation (106) d'un module de défibrillation (102) ;
le processeur (101a) est en outre configuré pour :
acquérir un signal d'électrocardiogramme lequel est détecté par l'unité de détection d'électrocardiogramme (105) ; et
commander le départ et l'arrêt d'une opération de défibrillation de module(s) de défibrillation (102) dans le mode de défibrillation cible, le(s)quel(s) module(s) correspond(ent) au mode de défibrillation cible, sur la base du signal d'électrocardiogramme acquis.

14. Dispositif médical avec fonction de défibrillation selon la revendication 13, **caractérisé en ce qu'**un nombre d'unité(s) de détection d'électrocardiogramme est d'un ou plus, et chaque unité de détection d'électrocardiogramme (105) peut détecter un canal de signal d'électrocardiogramme ;
lorsque de multiples unités de détection d'électrocardiogramme (105) existent, le processeur (101a) est en outre configuré pour :
acquérir de multiples canaux de signal d'électrocardiogramme lesquels sont acquis par les unités de détection d'électrocardiogramme (105) ;
réaliser un processus anti-interférence sur les multiples canaux de signal d'électrocardiogramme, de sorte à obtenir des données d'électrocardiogramme disponibles ; et
commander le départ et l'arrêt de l'opération de défibrillation du (des) module(s) de défibrillation (102) dans le mode de défibrillation cible, le(s)quel(s) module(s) correspond(ent) au mode de défibrillation cible, sur la base des données d'électrocardiogramme disponibles.

15. Dispositif médical avec fonction de défibrillation selon l'une quelconque des revendications 2 à 14, **caractérisé en ce que** le processeur (101a) est en outre configuré pour :
mesurer l'impédance d'un patient par une électrode de défibrillation (106) avant de démarrer le mode de défibrillation cible ; et
régler, en fonction de l'impédance mesurée du patient, une tension de charge de module(s) de défibrillation (102) le(s)quel(s) correspond(ent) au mode de défibrillation cible, de sorte à obtenir une intensité de courant de décharge laquelle intensité se trouve dans une plage préréglée ;
de préférence, chaque module de défibrillation (102) a de manière correspondante sa propre électrode de défibrillation (106a, 106b, 106c, 106d) ; lorsque le mode de défibrillation cible correspond à au moins deux modules de défibrillation (102a, 102b), les électrodes de défibrillation (106a, 106b, 106c, 106d) lesquelles correspondent à différents modules de défibrillation ont des couleurs différentes.
